# EUROPEAN PATENT APPLICATION

(11) **EP 2 047 856 A1**
(43) Date of publication of application: **15.04.2009**
(21) Application number: 07805892.2
(22) Date of filing: 02.08.2007
(51) Int. Cl.: A61K 35/20, A23L 1/30, A61K 31/661, A61P 31/16

(54) **AGENT FOR PREVENTING INFECTION**

(30) Priority: 04.08.2006 JP 2006213273; 04.08.2006 JP 2006213276
(71) Applicant: Snow Brand Milk Products Co., Ltd., Sapporo-shi Hokkaido 065-0043 (JP)
(72) Inventor: KAWAKAMI, Hiroshi, Saitama 350-1142 (JP)
(74) Representative: Schuster, Thomas
(86) International application number: PCT/JP2007/065171
(87) International publication number: WO 2008/016108

(57) **Abstract**

An agent for preventing an infection with an influenza virus or a food or drink for preventing infection with an influenza virus is provided. It is the agent for preventing an infection with an influenza virus and a food or drink for preventing an infection with an influenza virus, which comprises a fat globule membrane component as an active ingredient. It is the agent for preventing an infection with an influenza virus and a food or drink for preventing an infection with an influenza virus, which comprises a sphigosine-containing phospholipid and/or a derivative thereof, particularly sphingomyelin, as an active ingredient.

## Description

### TECHNICAL FIELD

The present invention relates to an agent for preventing infection with an influenza virus, which comprises a fat globule membrane component as an active ingredient.

Also, the invention relates to an agent for preventing infection with an influenza virus, which comprises a sphingosine-containing phospholipid and/or a derivative thereof, particularly sphingomyelin, as an active ingredient.

### BACKGROUND OF THE INVENTION

An influenza virus causes prevalence almost annually, by infecting people from the air. Though its threat has been declining in recant years due to an improvement of sanitation and progress in medical science, there still are cases of generating the dead.

There are three types of type A, type B and type C in the influenza virus, and among them, the type A virus is apt to generate mutation and apt to induce a world-wide flu epidemic. Prevention against the infection with an influenza virus is mainly carried out by an inoculation of vaccine. However, since the influenza virus is apt to cause antigenic shift, antigenic drift and the like mutations, the prevalent virus does not coincide with an antigen of the vaccine in many cases, so that it is the present situation that the effect of prevention by vaccine is not satisfactory.

Because of this, preventative inoculation of vaccine to children is also not under obligation now. Amantadine, Oseltamivir and the like are cited as the therapeutic agents for the influenza, but it is necessary to take care of their use because it is necessary to take side effects, development of resistant bacteria and the like problems into consideration. Based on such situations, concern has been directed toward an agent or food or drink which can be ingested daily and safely and is effective in preventing infection with an influenza virus.

### DISCLOSURE OF THE INVENTION

### PROBLEMS THAT THE INVENTION IS TO SOLVE

An object of the invention is to provide an agent for preventing infection with an influenza virus, which can be ingested daily and safely.

### MEANS FOR SOLVING THE PROBLEMS

By taking note of milk components having various physiological activities, the present inventors have conducted intensive studies on their preventive effect on the infection with the influenza virus and found the preventive effect on the infection with an influenza virus in a fat globule membrane component and a sphingosine-containing phospholipid and/or a derivative thereof, thus resulting in the accomplishment of the invention.

That is, the invention relates to the following.
(1) An agent for preventing an infection with an influenza virus, which comprises a fat globule membrane component as an active ingredient.
(2) An agent for preventing an infection with an influenza virus, which comprises a sphingosine-containing phospholipid and/or a derivative thereof as an active ingredient.
(3) The agent for preventing an infection with an influenza virus according to (3), wherein the sphingosine-containing phospholipid is sphingomyelin.
(4) A food or drink for preventing an infection with an influenza virus, which comprises a fat globule membrane component.
(5) A food or drink for preventing an infection with an influenza virus, which comprises a sphingosine-containing phospholipid and/or a derivative thereof.
(6) The food or drink for preventing an infection with an influenza virus according to (5), wherein the sphingosine-containing phospholipid is sphingomyelin.

### ADVANTAGE OF THE INVENTION

The infection with an influenza virus can be prevented by the agent for preventing infection with an influenza virus and food or drink for preventing infection with an influenza virus of the invention.

Since the agent for preventing infection with an influenza virus and food or drink for preventing infection with an influenza virus of the invention comprises a fat globule membrane component as an active ingredient, these can be provided in large amounts relatively inexpensively and also have a characteristic of markedly high safety.

In addition, since the agent for preventing infection with an influenza virus and food or drink for preventing infection with an influenza virus of the invention comprises a sphingosine-containing phospholipid and/or a derivative thereof, particularly sphingomyelin, as an active ingredient, these can be provided in large amounts relatively inexpensively and also have a characteristic of markedly high safety.

### BEST MODE FOR CARRYING OUT THE INVENTION

### (Fat globule membrane component)

The invention relates to an agent for preventing infection with an influenza virus, which comprises a fat globule membrane component as an active ingredient, and also relates to a food or drink provided with a preventative action on the infection with an influenza virus by blending with a fat globule membrane component.

The fat globule membrane component is a membrane which covers a milk fat globule secreted from the mammary gland and not only has a function to disperse fat into milk but also has many physiological functions as a food for new born animals. The milk fat globule membrane of a milk comprises about 45% by mass of a protein and about 55% by mass of a lipid, and contains a polymer glycoprotein called milk mucin as the protein. Also, about 70% by mass of triacylglycerol, about 27% by mass of a phospholipid and about 3% by mass of a cholesterol and the like are contained as the lipid.

Regarding the milk fat globule membrane of a milk, protection of milk globule membrane, stabilization of milk fat emulsion, acceleration of lipid digestion, protection from infection with specific bacteria and the like functions are known. On the other hand, it is known that a sialic acid-highly containing mucin isolated from a mucous tissue of an animal has an anti-influenza virus activity (Biochem. J., 277, 713 - 718, 1991), but its composition components is completely different from the milk fat globule membrane components according to the invention.

As the fat globule membrane component to be used in the invention, for example, a butter milk which is obtained when butter granules are produced by treating a cream obtained by centrifuging milk of a cow or the like mammal with a churn or the like may be used directly.

Also, for example, by producing butter granules by treating a cream, from which fat-free milk components were removed by repeating several times of a process in which the aforementioned cream is mixed by adding the same amount of water and centrifuged to prepare a cream having an original fat ratio, with a churn or the like, the thus obtained butter milk may be used as the fat globule membrane component.

In addition, a butter serum which is obtained as a residue when butter oil is produced by heating and centrifuging the aforementioned butter granules may be used as the fat globule membrane component.

Thus, since sufficient amount of the fat globule membrane component is contained in these butter milk and butter serum, these butter milk and butter serum may be used directly as the fat globule membrane component, but these butter milk and butter serum may be used by increasing purity of the fat globule membrane component by further purifying by dialysis, ammonium sulfate fractionation, gel filtration, isoelectric precipitation, ion exchange chromatography, solvent fractionation, ultrafiltration (UF), microfiltration (MF) and the like methods.

For example, in order to obtain a composition having an increased content of the fat globule membrane component, the separation filtration techniques by a UF membrane or MF membrane can be used. Since the lipid fraction can not permeate the UF membrane or MF membrane, proteins, lactose and minerals can be removed.

Fractionating molecular weight of the UF membrane and pore size of the MF membrane are not so strict, and those skilled in the art can set appropriate values based on tests. For example, in the case of MF, a tentative standard of the pore size is 1.2 µm or less, preferably 0.2 µm or less, and in the case of UF, a tentative standard of the fractionating molecular weight is 10,000 or more, preferably from 50,000 to 100,000.

The filtered concentrate may be spray-dried to prepare a composition having an increased content of the fat globule membrane component, or the UF membrane- or MF membrane-treated concentrate may be homogenized (100 kg/cm² or more) and then MF- or UF-treated and spray-dried to prepare a composition having a further increased content of the fat globule membrane component.

Crude fraction of the fat globule membrane component can be obtained, for example, in the following manner. Crude fat is extracted by treating the composition having an increased content of the fat globule membrane component with ethanol, methanol or the like polar solvent and a combination of a non-polar solvent with a polar solvent, such as ether-ethanol (1:3 v/v), chloroform-methanol (2:1 v/v), chloroform-methanol-water (1:2:0.8 v/v) or the like. By fractionating this crude fat with acetone, an acetone-insoluble fraction rich in the fat globule membrane component can be obtained.

Also, butter serum which is the residue of the preparation of butter oil from cream contains the fat globule membrane component in a large amount and therefore is one of the desirable materials. By extracting a crude fat from this butter serum with ethanol, a product obtained by fractionating this crude fat with acetone may be used. In addition, an extraction fraction of a mixed solvent of hexane-ethanol-water (JP-A-7-173182) can also be used. Further, a fat globule membrane component derived from a commercially available milk may also be used.

Regarding blending amount of the fat globule membrane component in the agent for preventing infection with an influenza virus and food or drink for preventing infection with an influenza virus of the invention, in the case of an adult, the blending amount of the fat globule membrane component may be adjusted in such a manner that the fat globule membrane component can be ingested in an amount of approximately from 0.1 mg to 5000 mg per day. By setting within this range, the preventive action on the infection with an influenza virus can be exerted. Since the fat globule membrane component as the active ingredient of the agent for preventing infection with an influenza virus of the invention is a milk component, it can be said that there is no problem regarding its safety even when ingested in a large amount.

### (Sphingosine-containing phospholipid)

Also, the invention relates to an agent for preventing infection with an influenza virus, which comprises a sphingosine-containing phospholipid and/or a derivative thereof, particularly sphingomyelin, as an active ingredient, and also relates to a food or drink provided with a preventative action on an infection with an influenza virus by blending with a sphingosine-containing phospholipid and/or a derivative thereof, particularly sphingomyelin.

In spite of the presence of sphingomyelin in a milk in a large amount of from 20 to 30% by mass in phospholipid, studies on its function are limited to cell levels, and information on its physiological functions in a living body is extremely scarce. Accordingly, its effectiveness as a component of nutrient substances has not been recognized.

Regarding applications of sphingomyelin, antiinflammatory and analgesic external preparations, an agent for improving digestion absorption functions of lipid, an agent for treating intestinal movement function insufficiency diseases (JP-A-5-186330, JP-A-11-269074, JP-A-2003-252765) and the like are known, but nothing has been revealed on its preventive effect on the infection with an influenza virus so that it has not been used for the purpose of preventing infection with an influenza virus.

On the other hand, cases of using sphingomyelin as a lipid component for the purpose of forming liposome by emulsifying a component having an anti-viral action (JP-T-2006-508045) (the term "JP-T" as used herein means a published Japanese translation of a PCT patent application) and an antigen to be used as vaccine (JP-A-5-339169) have been reported, but it is not known that the sphingomyelin itself has anti-influenza virus activity.

In addition, it is conventionally known that ganglioside which is a glycolipid contained in a milk has an anti-influenza activity (JP-A-4-105616), but sialic acid which is a constituent component is important for this action which is a function expressed by a mechanism in which a glycolipid comprising sialic acid competitively inhibits binding of the virus with mucous epithelium. Accordingly, it is evident that it does not remind of a function of sphingomyelin which does not comprise sialic acid.

The sphingosine-containing phospholipid and/or a derivative thereof, particularly sphingomyelin, to be used in the invention may be purified or may be used as a sphingomyelin-containing phospholipid.

Though sphingomyelin is richly contained in an animal brain and a milk fat, a milk-derived one is desirable from the viewpoint of carrying out the invention. As the milk-derived sphingomyelin, it is desirable to use a raw milk, whey protein concentrate (WPC) and the like as a material.

As the method for obtaining a sphingomyelin-containing phospholipid fraction from the raw milk, WPC and the like, a method for extracting with ether or acetone (JP-A-3-47192), a method for using a water-soluble fraction containing butter curd or butter serum obtained by heat-melting butter, and the like conventionally known methods can be exemplified. The sphingomyelin content of the fraction obtained by employing these materials and methods is about 28% by mass and about 9% by mass, respectively.

In addition, sphingomyelin having increased purity can be obtained by purifying the aforementioned sphingomyelin-containing phospholipid fraction by dialysis, ammonium sulfate fractionation, gel filtration, isoelectric precipitation, ion exchange chromatography, solvent fractionation, ultrafiltration (UF), microfiltration (MF) and the like techniques.

These sphingomyelin and sphingomyelin-containing phospholipid can optionally take a liquid, a powder, a tablet and the like forms and can be orally administered directly. In addition, not only sphingomyelin but also a phospholipid composition containing an effective amount of phosphatidylcholine defined by the human nutrition requirements may be used.

Regarding the blending amount of the sphingosine-containing phospholipid and/or a derivative thereof in the agent for preventing infection with an influenza virus and food or drink for preventing infection with an influenza virus of the invention, in the case of adults, the blending amount and the like may be adjusted in such a manner that the sphingosine-containing phospholipid and/or a derivative thereof, particularly sphingomyelin, can be ingested in an amount of approximately from 0.1 mg to 5000 mg per day. By setting within this range, the preventive action on the infection with an influenza virus can be exerted. Since the sphingosine-containing phospholipid, particularly sphingomyelin, which is the active ingredient of the agent for preventing infection with an influenza virus of the invention is a milk component, it can be said that there is no problem regarding its safety even when ingested in a large amount.

As the dosage form of the agent for preventing infection with an influenza virus of the invention, for example, tablets, capsules, granules, powdered materials, powders, solutions and the like can be exemplified. These may be orally administered or may be transnasally administered. In addition, these dosage forms can be produced by conventionally known general methods. For example, they are formed by mixing with pharmaceutical preparation production-acceptable carriers, fillers and the like.

In addition, as the food or drink of the invention provided with the preventing action on the infection with an influenza virus, for example, those in which the fat globule membrane component or the phospholipid and/or a derivative thereof is blended with a milk, milk drink, coffee drink, juice, jelly, biscuit, bread, noodle, sausage and the like food and drink or various types of powdered milk, as well as nutritious compositions intended for sucklings, babies, low birth weight babies and the like, can be exemplified. Since it is possible to ingest these in the usual way, the infection with an influenza virus can be prevented.

The following describes the invention further in detail with reference to examples and test examples, but these are merely illustrations and the invention is not limited thereto.

### Example 1

Cream adjusted to have a fat ratio of 40% by mass was treated with a churn and separated into butter granules and butter milk. Thereafter, a fat globule membrane component was obtained by freeze-drying the thus obtained butter milk.

### Example 2

An operation, in which the same amount of water was added to the cream adjusted to have a fat ratio of 40% by mass and mixed and cream having a fat ratio of 40% by mass was prepared by a centrifuge, was repeated three times to remove fat-free milk components from the cream, and then this cream was treated with a churn and separated into butter granules and butter milk. Thereafter, a fat globule membrane component was obtained by freeze-drying the thus obtained butter milk.

### Example 3

An operation, in which the same amount of water was added to the cream adjusted to have a fat ratio of 40% by mass and mixed and cream having a fat ratio of 40% by mass was prepared by a centrifuge, was repeated three times to remove fat-free milk components from the cream, and then this cream was treated with a churn and separated into butter granules and butter milk. Next, this butter milk was treated overnight with 50% saturation ammonium sulfate and then centrifuged to collect the supernatant. Thereafter, the thus obtained supernatant was dialyzed with water and then freeze-dried to obtain a highly concentrated fat globule membrane fraction.

### [Test Example 1]

### (Verification of the effect of oral administration of fat globule membrane component on the prevention of infection with an influenza virus)

A mouse (Balb/c, male, 6 weeks of age) was infected with 1 x 10³ pfu in viral quantity of influenza virus PR 8 (H1N1). Before the infection treatment, the fat globule membrane component obtained in Example 2 was orally administered, and its infection preventive effect was judged by the virus titer in the nasal cavity washes 3 days after the virus infection. In carrying out the oral administration, the fat globule membrane component was used by dissolving its powder in distilled water. A plaque method which uses MDCK cell was used in the judgment.

The results are shown in Table 1. The nasal cavity virus titer was lowered with significance in the fat globule membrane component administration group. This indicates effect of the fat globule membrane component to prevent infection with an influenza virus.

**[Table 1]**

| Sample | Nasal cavity virus titer x (PFU/ml: 10^{x}) | |
|---|---|---|
| Control (no administration) | | 2.91 ± 0.07 |
| Fat globule | 1 mg | 2.79 ± 0.31 |
| membrane | 10 mg | 2.41 ± 0.12 |
| component | 100 mg | 2.19 ± 0.17 |

### EXAMPLE 4

Solutions for intra-nasal cavity spray were produced by dissolving 5 g of the highly concentrated fat globule membrane fraction obtained in Inventive Example 3 in 200 ml of distilled water for injection.

### EXAMPLE 5

The materials of the formulation shown in Table 2 were mixed, made into granules and then filled in capsules, thereby producing capsules for preventing infection with an influenza virus.

**[Table 2]**

| | |
|---|---|
| Highly concentrated fat globule membrane fraction (Example 3) | 20.0 (% by mass) |
| Lactose | 24.5 |
| Soluble starch | 55.0 |
| Magnesium stearate | 0.5 |

### Example 6

The materials of the formulation shown in Table 3 were mixed, filled in a container and then heat-sterilized, thereby producing a food or drink for preventing infection with an influenza virus.

**[Table 3]**

| | |
|---|---|
| Highly concentrated fat globule membrane fraction (Example 3) | 2.5 (% by mass) |
| Sucrose | 7.5 |
| Citric acid | 0.6 |
| Apple juice | 10.0 |
| Water | 79.4 |

### Example 7

A reaction liquid obtained by allowing a protease to act upon a 10% by mass aqueous solution of a whey protein concentrate (WPC) was extracted with a chloroform-methanol (2:1) solution, concentrated and further extracted with acetone to obtain a complex lipid fraction. Next, this complex lipid fraction was treated with a fluorosilyl column chromatography and extracted stepwise with chloroform-methanol solutions to obtain a phospholipid fraction. This phospholipid fraction was treated with a silica gel chromatography and extracted stepwise with chloroform-methanol solutions, and the result was freeze-dried to obtain sphingomyelin. This sphingomyelin was treated with a thin layer chromatography and then color-developed with Dittmer's reagent and measured by a densitometry to find that the sphingomyelin content was 95.2% by mass. It is possible to use the sphingomyelin obtained in this manner directly as an agent for preventing infection with an influenza virus.

### [Test Example 2]

### (Verification of the effect to prevent infection with type A influenza virus and type B influenza virus)

Mice (Balb/c, male, 6 weeks of age) were transnasally infected with A/Guinzhou virus as the type A influenza virus or B/Ibaraki virus as the type B influenza virus, and at the same time, a 100 µg/ml solution of the sphingomyelin (SPM) obtained in Example 1 was transnasally administered at a dose of 5 µl/nasal cavity dose (dose: 0.5 µg), and the preventive effect on the infection with an influenza virus was judged by the virus titer in the nasal cavity washes. In this connection, groups transnasally infected with respective influenza viruses alone were prepared. A plaque method which uses MDCK cell was used in the judgment.

The results are shown in Table 4. As a result of this, infection preventive effect by the administration of sphingomyelin was confirmed for both of the type A and type B. Particularly, more considerable effect was confirmed for the type A influenza virus.

**[Table 4]**

| | Sample | Nasal cavity virus titer |
|---|---|---|
| A/Guizhou | Control | 2.93 ± 0.08 |
| virus | SPM | 1.99 ± 0.31 |
| | | |
| B/Ibaraki | Control | 2.91 ± 0.12 |
| virus | SPM | 2.73 ± 0.06 |

### [Test Example 3]

### (Verification of the effect of oral administration of sphingomyelin on the prevention of infection with an influenza virus)

A mouse (Balb/c, male, 6 weeks of age) was infected with 1 x 10³ pfu in viral quantity of influenza virus PR 8 (H1N1). Before the infection treatment, sphingomyelin was orally administered, and its infection preventive effect was judged by the virus titer in the nasal cavity washes 3 administration, sphingomyelin was used by dispersing in water. A plaque method which uses MDCK cell was used in the judgment.

The results are shown in Table 5. The nasal cavity virus titer was lowered with significance in the sphingomyelin administration group. This indicates the effect of sphingomyelin to prevent infection with an influenza virus.

**[Table 5]**

| Sample | Nasal cavity virus titer x (PFU/ml: 10^{x}) |
|---|---|
| Control (no administration) | 2.91 ± 0.07 |
| | |
| Sphingomyelin administration 1 mg | 2.23 ± 0.16 |
| Sphingomyelin administration 10 mg | 2.15 ± 0.29 |

### Example 8

Solutions for intra-nasal cavity spray were produced by dissolving 5 g of the sphingomyelin obtained in Example 7 in 200 ml of distilled water for injection.

### Example 9

The materials of the formulation shown in Table 6 were mixed, made into granules and then filled in capsules, thereby producing capsules for preventing infection with an influenza virus.

**[Table 6]**

| | |
|---|---|
| Sphingomyelin (Example 7) | 20.0 (% by mass) |
| Lactose | 24.5 |
| Soluble starch | 55.0 |
| Magnesium stearate | 0.5 |

### Example 10

The materials of the formulation shown in Table 7 were mixed, filled in a container and then heat-sterilized, thereby producing a food or drink for preventing infection with an influenza virus.

**[Table 7]**

| | |
|---|---|
| Sphingomyelin (Example 7) | 2.5 (% by mass) |
| Sucrose | 7.5 |
| Citric acid | 0.6 |
| Apple juice | 10.0 |
| Water | 79.4 |

While the invention has been described in detail and with reference to specific embodiments thereof, it will be apparent to one skilled in the art that various changes and modifications can be made therein without departing from the spirit and scope of the invention.
This application is based on two Japanese patent applications filed on August 4, 2006 (Japanese Patent Application No. 2006-213276, Japanese Patent Application No. 2006-213273), the entire contents thereof being thereby incorporated by reference.

## Claims

1. An agent for preventing an infection with an influenza virus, which comprises a fat globule membrane component as an active ingredient.

2. An agent for preventing an infection with an influenza virus, which comprises a sphingosine-containing phospholipid and/or a derivative thereof as an active ingredient.

3. The agent for preventing an infection with an influenza virus according to claim 3, wherein the sphingosine-containing phospholipid is sphingomyelin.

4. A food or drink for preventing an infection with an influenza virus, which comprises a fat globule membrane component.

5. A food or drink for preventing an infection with an influenza virus, which comprises a sphingosine-containing phospholipid and/or a derivative thereof.

6. The food or drink for preventing an infection with an influenza virus according to claim 5, wherein the sphingosine-containing phospholipid is sphingomyelin.
